(19) Europäisches Patentamt
European Patent Office
Office européen des brevets

(11) Publication number: **0 148 094**
**A2**

# (12) EUROPEAN PATENT APPLICATION

(21) Application number: 84420219.2

(22) Date of filing: 26.12.84

(51) Int. Cl.[4]: C 07 D 407/04
A 61 K 31/365

(30) Priority: 27.12.83 JP 244787/83

(43) Date of publication of application:
10.07.85 Bulletin 85/28

(84) Designated Contracting States:
DE FR GB IT

(71) Applicant: Kochi, Mutsuyuki
19, Matsudo Shinden
Matsudo-shi Chiba-ken(JP)

(71) Applicant: MEIJI SEIKA KAISHA LTD.
4-16 Kyobashi 2-chome
Chuo-ku Tokyo 104(JP)

(72) Inventor: Kochi, Mutsuyuki
19, Matsudo Shinden Matsudo-shi
Shiba-ken(JP)

(72) Inventor: Ito, Teiichiro
37-14, Hakusan 4-chome
Bunkyo-ku Tokyo(JP)

(72) Inventor: Hisamatsu, Takashi
1-11-22, Ohkubo, Kohnan-ku
Yokohama-shi Kanagawa-ken(JP)

(74) Representative: Maureau, Pierre et al,
Cabinet GERMAIN & MAUREAU Le Britannia - Tour C 20,
Boulevard E. Déruelle
F-69003 Lyon(FR)

(54) **Anti-tumor agent comprising an O-benzylidene-L-ascorbic acid or a salt thereof, and the production of the latter compound.**

(57) A known compound, 5,6-O-benzylidene-L-ascorbic acid and a new alkali metal or alkaline earth metal salt thereof have now been found to exhibit anti-tumor activity and be effective as anti-tumor agent. 5,6-O-benzylidene-L-ascorbic acid may efficiently be produced by a new process comprising reacting L-ascorbic acid with α,α-dimethoxytoluene in the presence of an acid catalyst such as p-toluene-sulfonic acid.

EP 0 148 094 A2

0148094

## SUMMARY OF THE INVENTION

This invention relates to a new anti-tumor agent comprising as active ingredient at least one of 5,6-O-benzylidene-L-ascorbic acid and a metal salt thereof. This invention also relates to a process for the production of 5,6-O-benzylidene-L-ascorbic acid or a metal salt thereof.

## BACKGROUND OF THE INVENTION

Japanese patent application first publication "KOKAI" No. 70428/79 describes that an anti-tumor activity is shown by aromatic aldehydes and derivatives thereof, particularly fluorobenzaldehydes, phthalaldehydes, mono-benzalpentaerythritol, 4,6-O-benzylidene-D-glucopyranose, N-benzylideneethylamine, and other some various benzylidene compounds. However, the particular compounds of anti-tumor activity which are specifically mentioned in said Japanese patent application first publication "KOKAI" No. 70428/79 are all sparingly soluble in water, so that they must be dissolved in a large volume of water for their administration by intravenous injection and that they are not always easy to be utilized in practice. While, some researchers have alleged that L-ascorbic acid (vitamin C) exhibits an anti-tumor activity, and this was tried to be demonstrated by test. However, it has not yet been recognized that L-ascorbic acid exhibits an anti-tumor activity which is high enough to be used generally as anti-tumor agent for therapeutic treatment of tumors in clinics.

## DETAILED DESCRIPTION OF THE INVENTION

We, the present inventors, have paid attention on that an O-benzylidene derivative of sugar being the 4,6-O-benzylidene-D-glucopyranose which is disclosed in the above-mentioned Japanese patent application first publication shows the anti-tumor activity. With getting an idea from the above-mentioned facts, we have made extensive researches on the synthesis of various L-ascorbic acid derivatives and discovery of the anti-tumor activity of said derivatives in an attempt to provide such L-ascorbic acid derivatives which not only exhibit an effective anti-tumor activity but also are soluble in water. As a result, we have now found that 5,6-O-benzylidene-L-ascorbic acid and metal salts thereof, particularly alkali metal and alkaline earth metal salts thereof which are now synthesized by us show an anti-tumor activity, and that the metal salts of 5,6-O-benzylidene-L-ascorbic acid are readily soluble in water.

According to one aspect of this invention, therefore, there is provided an anti-tumor agent which comprises as active ingredient at least one of 5,6-O-benzylidene-L-ascorbic acid and metal salts thereof. In the anti-tumor agent or composition of this invention, the active ingredient compound may be in association with a known pharmaceutically acceptable carrier, either solid or liquid.

The active ingredient compound used according to this invention, that is, the 5,6-O-benzylidene-L-ascorbic

acid is a known compound reported in the "Steroids" 12, 309 (1968) and is represented by the formula;

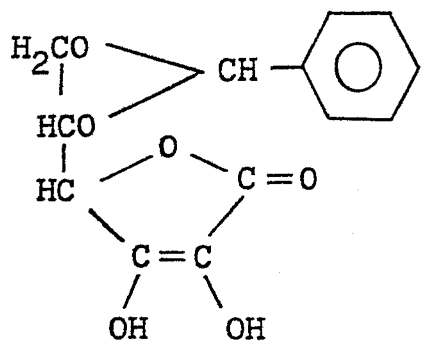

and this compound is a white colored crystalline compound which is sparingly soluble in water. While, the metal salts of 5,6-O-benzylidene-L-ascorbic acid are readily soluble in water, and their aqueous solutions show a pH value of nearly 7.0 and display excellent properties to be advantageously administered in the form of injectable solution. Especially, the 5,6-O-benzylidene-L-ascorbic acid alkali metal salts and alkaline earth metal salts mentioned above are novel compounds which have not been disclosed in any previous documents, and which are readily soluble in water. Among them, the sodium salt is particularly suitable for formulation into an anti-tumor agent for injection.

According to the method of the above-mentioned document "Steroid" 12, 309 (1968), the preparation of 5,6-O-benzylidene-L-ascorbic acid was effected by reacting L-ascorbic acid with benzaldehyde and zinc chloride. However, this prior art method requires a long reaction time of

45 hours and gives disadvantageously a very poor yield of the 5,6-O-benzylidene-L-ascorbic acid which is as low as 12%. We have now found that when L-ascorbic acid is reacted with α,α-dimethoxytoluene, 5,6-O-benzylidene-L-ascorbic acid may be obtained in a favorable yield of 70% or above, only with needing a reaction time of about 4 hours.

According to another aspect of this invention, therefore, there is provided a process for the production of 5,6-O-benzylidene-L-ascorbic acid and a metal salt thereof, which process comprises reacting L-ascorbic acid with α,α-dimethoxytoluene to produce 5,6-O-benzylidene-L-ascorbic acid, and if desired, further reacting the latter compound with an alkaline compound of a metal, particularly an alkali metal or alkaline earth metal in a manner known per se, to give a corresponding metal salt of 5,6-O-benzylidene-L-ascorbic acid.

According to the process of this invention, the reaction of L-ascorbic acid with α,α-dimethoxytoluene may be achieved in the presence of an acid catalyst such as p-toluenesulfonic acid under heating and in an inert organic solvent such as dimethylformamide, dimethylsulfoxide and the like. The 5,6-O-benzylidene-L-ascorbic acid as produced may then be recovered from the reaction solution in a conventional manner.

The free 5,6-O-benzylidene-L-ascorbic acid may be converted into a metal salt thereof, by reacting with an

alkaline compound of a metal in a conventional manner.

The preparation of the metal salts of 5,6-O-benzylidene-L-ascorbic acid may conveniently be performed by suspending white crystals of 5,6-O-benzylidene-L-ascorbic acid in water, and adding to the aqueous suspension an equi-molar proportion of an alkaline metal compound such as alkaline sodium compound, for example sodium hydrogen carbonate, followed by stirring the mixture, so that the crystals may dissolve in water and undergoes the salt-forming reaction. The resulting reaction solution may then be either lyophilized or added with an organic solvent such as isopropanol in which the sodium salt of 5,6-O-benzylidene-L-ascorbic acid is sparingly soluble, to deposit sodium salt of 5,6-O-benzylidene-L-ascorbic acid as white to pale pink colored crystals. Similarly, the potassium salt of 5,6-O-benzylidene-L-ascorbic acid may be obtained by reaction of 5,6-O-benzylidene-L-ascorbic acid with potassium hydrogen carbonate, and the calcium salt of 5,6-O-benzylidene-L-ascorbic acid by reaction of 5,6-O-benzylidene-L-ascorbic acid with 1/2 molar proportion of calcium carbonate.

From some animal experiments, we have found that 5,6-O-benzylidene-L-ascorbic acid and alkali metal and alkaline earth metal salts thereof exhibit an anti-tumor activity and are substantially non-toxic to animals.

The anti-tumor agent according to this invention

may be administered orally and parenterally. For the oral administration, the anti-tumor agent may be given in the form of capsule, tablet, granule or powder. For the parenteral administration, the anti-tumor agent may be given in the form of injection, intravenous drip or suppository.

The content of the active ingredient in the anti-tumor agent or composition according to this invention may vary depending on the form of the agent. It may usually be about 0.1 to 20% by weight for oral administration and for absorption through mucous membrane, and about 0.01 to 10% by weight for parenteral administration.

The active ingredient compound used in the anti-tumor agent according to this invention may be formulated in a conventional manner into subcutaneous or intravenous injections, for example, into the form of aqueous solutions or suspensions in water or oil. The anti-tumor agent may also be formulated into the form of capsule, tablet, powder and the like for oral administration.

The active ingredient compound of the anti-tumor agent according to this invention may be formulated in association with a pharmaceutically acceptable carrier, optionally together with a surface active agent, an excipient, a lubricant and other suitable additives.

Appropriate dosage of the anti-tumor agent according to this invention may vary dependently upon the curative

effect to be attained and also upon the time period of therapeutic treatment with the anti-tumor agent. It is usually 500~1000 mg of the active ingredient compound for adult per day.

This invention will be illustrated in more detail with reference to the following Examples showing the production of the active compound and the experiments for controlling tumors.

Example 1

Production of 5,6-O-benzylidene-L-ascorbic acid;

L-Ascorbic acid (20 g), α,α-dimethoxytoluene (20 g) and a catalytic quantity (160 mg) of p-toluene-sulfonic acid were dissolved in 60 mℓ of dimethylformamide. The solution obtained was heated at 55~60°C for 4 hours to effect the reaction. The reactor used for carrying out the reaction was equipped with a reflux-condenser connected to an aspirator, and was evacuated during the reaction so that the methanol as produced from the reaction could be removed out of the reaction mixture. When the reaction was completed, the reaction solution was distilled under reduced pressure to remove the solvent. The resulting syrupy residue was washed with petroleum ether and water, each three times. Then, water and hexane were added to the washed syrup and the mixture was stirred to precipitate a crystalline product of 5,6-O-benzylidene-L-ascorbic acid which was then removed by filtration and dried to give 21.5 g of white colored

crystals of 5,6-O-benzylidene-L-ascorbic acid (72% yield). The product thus obtained was nearly pure substance but was further purified by recrystallization from methanol or benzene. The final pure product showed mp. of 166-168°C.

Example 2

5,6-O-Benzylidene-L-ascorbic acid (20 g) was suspended in 320 mℓ of water, to which was then added a powder of sodium hydrogen carbonate (6.4 g), so that the whole mixture became a clear solution after bubbling. The solution obtained was lyophilized to afford a sodium salt of 5,6-O-benzylidene-L-ascorbic acid as a white colored powder (yield 20 g). This sodium salt of 5,6-O-benzylidene-L-ascorbic acid showed the following physico-chamical properties:

(i) Readily soluble in water, (ii) Specific optical rotation: $(\alpha)_D^{20}$ + 24° (c, 1.5, $H_2O$), (iii) Melting point: begins to soften near 95°C and decomposes at 130-135°C with bubbling. (iv) Elementary analysis:

Found: C 53.97, H 4.01 (%)

Calcd. for $C_{13}H_{11}O_6Na$: C 54.55, H 3.87 (%).

Example 3

5,6-O-Benzylidene-L-ascorbic acid (13.2 g) was suspended in water (300 mℓ), to which was then added potassium hydrogen carbonate (5 g), whereby the ascorbic acid was dissolved in water. The resulting aqueous solution was lyophilized to give a potassium salt of 5,6-O-benzylidene-

L-ascorbic acid (yield 12 g). The product was a white powdery substance which was readily soluble in water, begined to soften near 90°C and decomposed at 130~140°C with bubbling.

Example 4

5,6-O-Benzylidene-L-ascorbic acid (13.2 g) was suspended in water (300 mℓ), to which was then added calcium carbonate (5 g), whereby the ascorbic acid was dissolved in water. The resulting aqueous solution was freeze-dried to give a calcium salt of 5,6-O-benzylidene-L-ascorbic acid (yield 13 g). This product was a white powdery substance which was readily soluble in water and decomposed near 200°C.

Experiment 1

Tests for anti-tumor activity were conducted using BALB/c-strain mice (male, 5-weeks-aged, 6 mice in each group). Fibro-sarcoma Meth-A cells (mouse solid tumor) were subcutaneously implanted into the axillary site in mice at a dosage of $2 \times 10^6$ cells/mouse. Starting on the next day subsequent to the implantation with fibro-sarcoma Meth-A cells, a solution of 5,6-O-benzylidene-L-ascorbic acid in olive oil or an aqueous solution of sodium salt of 5,6-O-benzylidene-L-ascorbic acid in distilled water to be tested was intraperitoneally administered to mice once a day for consecutive 9 days. Thirty days after the implantation with fibro-sarcoma Meth-A cells, the tumor was taken out of

the mice and the tumor volume was measured. Rate (%) of inhibition by the test compounds was calculated according to the following equation:

$$\text{Percentage of inhibition} = \frac{\text{(mean vol. of tumor in untreated animals)} - \text{(mean vol. of tumor in animals as treated with test compound)}}{\text{(mean vol. of tumor in untreated animals)}} \times 100$$

The results as obtained are shown in the following table:

TABLE

| Test Compound: 5,6-O-benzylidene-L-ascorbic acid | Dose (mg/kg/day) | Inhibition (%) |
|---|---|---|
| Sodium salt | 12.5<br>6.25 | 42.2<br>20.7 |
| Free acid | 12.5<br>6.25 | 40.3<br>19.5 |
| Control (untreated) | 0 | 0 |

What is claimed is:

1. An anti-tumor agent which comprises as active ingredient at least one of 5,6-0-benzylidene-L-ascorbic acid and metal salts thereof.

2. The agent as claimed in Claim 1 in which sodium salt, potassium salt or calcium salt of 5,6-0-benzylidene-L-ascorbic acid is used as the active ingredient compound.

3. The agent as claimed in Claim 1 in which the active ingredient compound has been formulated in association with a pharmaceutically acceptable carrier for the active ingredient.

4. A process for the production of 5,6-0-benzylidene-L-ascorbic acid or a metal salt thereof, which process comprises reacting L-ascorbic acid with α,α-dimethoxytoluene to produce 5,6-0-benzylidene-L-ascorbic acid, and if desired, reacting the latter compound with an alkaline compound of a metal particularly an alkali metal or alkaline earth metal in a known manner to produce an alkali or alkaline earth metal salt of 5,6-0-benzylidene-L-ascorbic acid.

5. A process as claimed in Claim 4 in which L-ascorbic acid is reacted with α,α-dimethoxytoluene in the presence of catalytic quantity of p-toluene-sulfonic acid in dimethylformamide under heating.